# EUROPÄISCHE PATENTSCHRIFT

(11) **EP 3 596 033 B1**
(45) Veröffentlichungstag und Bekanntmachung des Hinweises auf die Patenterteilung: **12.05.2021**
(21) Anmeldenummer: 18709023.8
(22) Anmeldetag: 12.03.2018
(51) Int. Cl.: C07C 7/04, C07C 7/08, C07C 11/167

(54) **VEREINFACHTES VERFAHREN ZUR GEWINNUNG VON REIN-1,3-BUTADIEN**
SIMPLIFIED METHOD FOR OBTAINING PURE 1,3-BUTADIENE
PROCÉDÉ SIMPLIFIÉ D'OBTENTION DE 1,3-BUTADIÈNE PUR

(30) Priorität: 13.03.2017 EP 17160642
(43) Veröffentlichungstag der Anmeldung: 22.01.2020
(73) Patentinhaber: BASF SE, 67056 Ludwigshafen am Rhein (DE)
(72) Erfinder: HEIDA, Bernd, 67158 Ellerstadt (DE); KELLER, Tobias, 67056 Ludwigshafen (DE)
(86) Internationale Anmeldenummer: PCT/EP2018/056050
(87) Internationale Veröffentlichungsnummer: WO 2018/166961

(56) Entgegenhaltungen:
- EP-A2- 0 284 971
- WO-A1-2013/083536
- WO-A2-2005/009931
- DE-A1- 10 105 660

## Beschreibung

Die vorliegende Erfindung betrifft ein Verfahren zur Gewinnung von Rein-1,3-Butadien aus einem Roh-C₄-Schnitt.

1,3-Butadien wird großtechnisch in der Regel aus so genannten C₄-Schnitten gewonnen, d.h. aus Gemischen von Kohlenwasserstoffen, worin die C₄-Kohlenwasserstoffe, insbesondere 1-Buten, i-Buten sowie 1,3-Butadien überwiegen.

C₄-Schnitte werden beispielsweise bei der Herstellung von Ethylen und Propylen durch thermisches Spalten, üblicherweise in Steamcrackern, insbesondere Naphtha- oder Gas-Crackern, erhalten. Weiterhin werden 1,3-Butadien enthaltende C₄-Schnitte bei der katalytischen Dehydrierung von n-Butan und/oder n-Buten erhalten. Als Ausgangsgasgemisch für die oxidative Dehydrierung von n-Butenen zu 1,3-Butadien kann jedes beliebige n-Butene enthaltende Gemisch benutzt werden. n-Butene enthaltende Gasgemische, die als Ausgangsgas in der oxidativen Dehydrierung von n-Butenen zu 1,3-Butadien eingesetzt werden, können durch nicht-oxidative Dehydrierung von n-Butan enthaltenden Gasgemischen hergestellt werden. Die 1,3-Butadien enthaltenden C₄-Schnitte werden nachfolgend als Roh-C₄-Schnitte bezeichnet. Sie enthalten neben geringen Mengen an C₃- und C₅-Kohlenwasserstoffen in der Regel Acetylene (Methylacetylen, Ethylacetylen und Vinylacetylen).

Es ist bekannt, dass Rein-1,3-Butadien aus Roh-C₄-Schnitten durch eine Abfolge bestimmter Verfahrensschritte gewonnen werden kann, bei denen aus dem Roh-C₄-Schnitt zunächst ein Roh-1,3-Butadien gewonnen und das Roh-1,3-Butadien dann weiter aufgereinigt wird, um daraus das Rein-1,3-Butadien zu gewinnen. Roh-1,3-Butadien ist ein Gemisch, das etwa 90 bis 99,5 Gew.-% 1,3-Butadien, insbesondere 98 bis 99 Gew.-% 1,3-Butadien, enthält. Die Spezifikationsanforderungen für Rein-1,3-Butadien sehen häufig einen Mindestgehalt an 1,3-Butadien von 99,6 Gew.-% und einen maximal zulässigen Gehalt an Acetylenen sowie an 1,2-Butadien von jeweils 20 ppm bezogen auf die Masse des Rein-1,3-Butadiens vor.

Die Gewinnung von 1,3-Butadien aus C₄-Schnitten ist wegen der geringen Unterschiede in den relativen Flüchtigkeiten der Komponenten eine komplexe Trennaufgabe. Daher führt man eine Extraktivdestillation durch, d.h. eine Destillation unter Zugabe eines selektiven Lösungsmittels, das einen höheren Siedepunkt als das aufzutrennende Gemisch aufweist und das die Unterschiede in den relativen Flüchtigkeiten der aufzutrennenden Komponenten erhöht. Das so erhaltene Roh-1,3-Butadien wird, um den Spezifikationsanforderungen zu entsprechen, destillativ zu Rein-1,3-Butadien aufgereinigt.

Den Verfahren zur Extraktivdestillation von C₄-Schnitten unter Verwendung von selektiven Lösungsmitteln ist gemeinsam, dass sich durch Gegenstromführung des aufzutrennenden C₄-Schnittes in Dampfform mit dem flüssigen selektiven Lösungsmittel bei geeigneten thermodynamischen Bedingungen, in der Regel bei niedrigen Temperaturen, meist im Bereich von 20 bis 80 °C und bei moderaten Drücken, häufig bei etwa 3 bis etwa 6 bar, das selektive Lösungsmittel mit den Komponenten aus dem C₄-Schnitt belädt, zu denen es eine höhere Affinität hat, wogegen die Komponenten, zu denen das selektive Lösungsmittel eine geringere Affinität hat, in der Dampfphase verbleiben und als Kopfstrom abgezogen werden. Aus dem beladenen Lösungsmittelstrom werden anschließend unter geeigneten thermodynamischen Bedingungen, d.h. bei höherer Temperatur und/oder niedrigerem Druck die Komponenten fraktioniert aus dem selektiven Lösungsmittel freigesetzt.

Beispielsweise wird gemäß der WO 2011/110562 A1 ein Roh-C₄-Schnitt selektiv hydriert, aus dem selektiv hydrierten C₄-Schnitt anschließend hochsiedende Bestandteile abgetrennt, der verbleibende C₄-Schnitt dann weiter durch Extraktivdestillation aufgearbeitet, um Roh-1,3-Butadien zu gewinnen. Das Roh-1,3-Butadien wird durch Reindestillation weiter zu Rein-1,3-Butadien aufgereinigt.

Die DE 101 05 660 offenbart ein Verfahren zur Gewinnung von Roh-1,3-Butadien durch Extraktivdestillation aus einem C₄-Schnitt mit einem selektiven Lösungsmittel. Das Verfahren wird in einer Trennwandkolonne (TK) durchgeführt, in der eine Trennwand (T) in Kolonnenlängsrichtung unter Ausbildung eines ersten Teilbereichs (A), eines zweiten Teilbereichs (B) und eines unteren gemeinsamen Kolonnenbereichs (C) angeordnet ist und der eine Extraktivwaschkolonne (K) vorgeschaltet ist.

Nach der WO 2013/083536 wird ein dampfförmiger gereinigter Roh-C₄-Schnitt als Einsatzstrom für eine Extraktivdestillation bereitgestellt, indem der flüssige Roh-C₄-Schnitt im oberen Drittel einer Destillationskolonne unter Ausbildung eines Verstärkungs- und eines Abtriebsteils zugeführt wird und aus der Destillationskolonne ein C₃-Kohlenwasserstoffe enthaltender Kopfstrom, ein C₄-Oligomere und -Polymere sowie die C₅₊-Kohlenwasserstoffe enthaltender Sumpfstrom und aus dem Abtriebsteil der dampfförmige gereinigte Roh-C₄-Schnitt als Seitenstrom abgezogen werden.

Die ältere europäische Patentanmeldung 17153120.5 offenbart ein Verfahren zur Gewinnung von Rein-1,3-Butadien aus einem Roh-C₄-Schnitt durch Extraktivdestillation mit einem selektiven Lösungsmittel. Das Verfahren bedient sich einer Vordestillationskolonne, worin eine C₃-Kohlenwasserstoffe enthaltende erste Leichtsiederfraktion als Kopfstrom, ein gasförmiger C₄-Schnitt als Seitenstrom und eine erste Hochsiederfraktion als Sumpfstrom abgezogen werden. Die Vordestillationskolonne ist durch eine in Längsrichtung der Vordestillationskolonne ausgerichtete Trennwand in einen Zulaufbereich und einen Seitenabzugsbereich unterteilt. Der gasförmige C₄-Schnitt wird mit einem selektiven Lösungsmittel in Kontakt gebracht, wobei man eine Butane und Butene enthaltende Kopffraktion und eine 1,3-Butadien und selektives Lösungsmittel enthaltende Sumpffraktion erhält. Aus der Sumpffraktion wird Roh-1,3-Butadien desorbiert. Das Roh-1,3-Butadien wird in einer Reindestillationskolonne von einer zweiten Hochsiederfraktion befreit.

1,3-Butadien ist eine polymerisationsfähige Verbindung und kann in verschiedenen Bereichen der Anlage unerwünschte polymere Ablagerungen bilden, die je nach Molekulargewicht und Vernetzungsgrad gummiartig oder spröde (so genannte Popcorn-Polymere) sein können. Die gummiartigen Beläge behindern den Wärmeübergang und führen zur Verringerung von Leitungsquerschnitten. Die Bildung von Popcorn-Polymeren kann schwere Schäden im Inneren der Anlage verursachen und zum Bersten von Kondensatoren und Leitungen führen. Die Ablagerungen müssen regelmäßig mit großem Aufwand und verlustreichen Stillstandzeiten aus Kolonnen und Rohrleitungen entfernt werden.

Die Errichtung und der Betrieb einer 1,3-Buteadienextraktionsanlage mit Vordestillationskolonne, Extraktions- und Ausgaserkolonnen und Reindestillationskolonne bedingen hohe spezifische Investitions- und Betriebskosten. In der Reindestillationskolonne besteht aufgrund der hohen 1,3-Butadienkonzentration ein hohes Sicherheitsrisiko durch die Bildung von Popcorn-Polymeren. Es ist daher wünschenswert, die Anlage und ihre Bedienung zu vereinfachen.

Die Aufgabe wird gelöst durch ein Verfahren zur Gewinnung von Rein-1,3-Butadien aus einem Roh-C₄-Schnitt, welches Rein-1,3-Butadien einen vorgegebenen Höchstgehalt an wenigstens einem Leichtsieder und einen vorgegebenen Höchstgehalt an 1,2-Butadien, jeweils bezogen auf 1,3-Butadien, aufweist, wobei man
a) vom Roh-C₄-Schnitt destillativ eine Leichtsiederfraktion und eine Hochsiederfraktion abtrennt, wobei man einen gereinigten C₄-Schnitt erhält, dessen Gehalt an dem wenigstens einen Leichtsieder, bezogen auf 1,3-Butadien, gleich oder niedriger ist als der vorgegebene Höchstgehalt an dem wenigstens einen Leichtsieder und dessen Gehalt an 1,2-Butadien, bezogen auf 1,3-Butadien, gleich oder niedriger ist als der vorgegebene Höchstgehalt an 1,2-Butadien;
b) den gereinigten C₄-Schnitt wenigstens einer Extraktivdestillation mit einem selektiven Lösungsmittel unterzieht, wobei man zumindest eine Butane und Butene enthaltende Fraktion und eine Rein-1,3-Butadien-Fraktion erhält.

Über die Leichtsiederfraktion werden die im Roh-C₄-Schnitt enthaltenden Leichtsieder entfernt, d.h. Komponenten, die (bei dem Betriebsdruck der Destillation in Schritt a)) einen niedrigeren Siedepunkt als 1,3-Butadien aufweisen. Leichtsieder sind insbesondere C₃-Kohlenwasserstoffe, wie Propan oder Propin (Methylacetylen). Vorzugsweise ist der wenigstens eine Leichtsieder Propin. Propin ist eine kritische C₃-Komponente, da etwaige andere C₃-Kohlenwasserstoffe, wie Popan, auch im Schritt b) zusammen mit dem Raffinat 1 bis unter die Nachweisgrenze entfernt werden würden. Es ist daher angestrebt, den Gehalt an Propin bereits in der Vordestillation auf einen Wert unterhalb der angestrebten Spezifikationsgrenze für Rein-1,3-Butadien zu senken. Der vorgegebene Höchstgehalt an Propin beträgt im Allgemeinen 20 ppm, vorzugsweise 10 ppm, bezogen auf 1,3-Butadien (Alle ppm-Angaben verstehen sich als Masse-ppm).

Da 1,3-Butadien und 1,2-Butadien ähnliche Löslichkeiten in selektiven Lösungsmitteln aufweisen, ist ihre Trennung durch Extraktivdestillation nicht ohne Weiteres möglich. Erfindungsgemäß wird der Gehalt an 1,2-Butadien im C₄-Schnitt daher bereits in einer Vordestillation auf einen Wert unterhalb der angestrebten 1,2-Butadien-Spezifikationsgrenze für Rein-1,3-Butadien gesenkt. 1,2-Butadien ist die Hochsiederkomponente im Roh-C₄-Schnitt, die die geringste Siedepunktsdifferenz zu 1,3-Butadien aufweist. In der Regel erfüllt ein C₄-Schnitt, der die 1,2-Butadien-Spezifikation erfüllt, ebenfalls die C₅₊-Kohlenwasserstoff-Spezifikation für 1,3-Butadien.

Der vorgegebene Höchstgehalt an 1,2-Butadien beträgt im Allgemeinen 25 ppm, insbesondere 15 ppm, bezogen auf 1,3-Butadien.

Oft weist das Rein-1,3-Butadien außerdem einen vorgegebenen Höchstgehalt an C₅₊-Kohlenwasserstoffen, bezogen auf 1,3-Butadien, auf und der Gehalt an C₅₊-Kohlenwasserstoffen des gereinigten C₄-Schnitts ist gleich oder niedriger als der vorgegebene Höchstgehalt an C₅₊-Kohlenwasserstoffen. Der vorgegebene Höchstgehalt an C₅₊-Kohlenwasserstoffen beträgt im Allgemeinen 20 ppm, vorzugsweise 10 ppm, bezogen auf 1,3-Butadien.

Über die Hochsiederfraktion werden die im Roh-C₄-Schnitt enthaltenden Komponenten entfernt, die einen höheren Siedepunkt als 1,3-Butadien aufweisen. 1,2-Butadien als

Schlüsselkomponente wird auf einen Gehalt unterhalb der Spezifikationsgrenze entfernt. C₅₊-Kohlenwasserstoffe und die im Roh-C₄-Schnitt enthaltenen C₄-Oligomere und -Polymere werden vollständig ausgeschleust. Über die Hochsiederfraktion werden auch Karbonyle wie Formaldehyd, Acetaldehyd, Propionaldehyd, n-Butyraldehyd, Crotonaldehyd, Aceton, Methylethylketon oder Acrolein, vollständig ausgeschleust. Da keine C₅₊-Komponenten, insbesondere sehr leicht polymerisierbare C₅-Diene wie Isopren oder cis-2-Pentadien aus der Vordestillation der Extraktivdestillation zugeführt werden, reichern sich diese im selektiven Lösungsmittel nicht an.

Die Abtrennung der Leichtsiederfraktion und der Hochsiederfraktion kann durch eine Abfolge von Destillationskolonnen erzielt werden, wobei in einer ersten Kolonne die Leichtsiederfraktion über Kopf abgetrennt wird und das Sumpfprodukt der ersten Kolonnen in einer zweiten Kolonne destilliert wird, wobei der gereinigte C₄-Schnitt über Kopf abgezogen wird und das Sumpfprodukt der zweiten Kolonne als Hochsiederfraktion abgezogen wird. Es ist auch möglich, zuerst Hochsieder und anschließend Leichtsieder abzutrennen.

Vorzugsweise erfolgt die Abtrennung der Leichtsiederfraktion und der Hochsiederfraktion jedoch in einer Trennwandkolonne oder einer thermisch gekoppelten Destillationskolonne. Beschreibungen des Aufbaus und der Funktion von Trennwandkolonnen und thermisch gekoppelten Kolonnen finden sich beispielsweise in Chem.-Ing.Techn. 61(1989) Nr. 2, S. 104-112 und in Gas Separation and Purification, 4(1990) Nr. 2, S. 109-114. Die Trennwandkolonne oder die thermisch gekoppelte Destillationskolonne erlaubt es, unter geringerem Energieaufwand ein Zulaufgemisch aus drei oder mehr Komponenten in praktisch reine Einzelkomponenten zu trennen. Obwohl in der Energiebilanz gleichwertig, weist die Trennwandkolonne gegenüber der thermisch gekoppelten Kolonne zusätzlich den Vorteil auf, dass hier anstelle zweier separater Destillationskolonnen eine einzige Kolonne eingesetzt wird.

Die Trennwandkolonne ist in einem mittleren Abschnitt durch eine im Wesentlichen in Längsrichtung der Vordestillationskolonne ausgerichtete Trennwand in einen Zulaufbereich und einen Seitenabzugsbereich unterteilt. Dabei führt man den flüssigen Roh-C₄-Schnitt in den Zulaufbereich ein und zieht den gasförmigen C₄-Schnitt aus dem Seitenabzugsbereich ab. Eine C₃-Kohlenwasserstoffe enthaltende Leichtsiederfraktion wird als Kopfstrom, eine Hochsiederfraktion als Sumpfstrom abgezogen.

Die Trennwandkolonne umfasst trennwirksame Einbauten, wie Trennböden, strukturierte Packungen oder regellose Schüttungen. Trennböden oder regellose Schüttungen sind bevorzugt. Bei den Trennböden verwendet man bevorzugt Glocken- und Ventilböden; bevorzugte Füllkörper für regellose Schüttungen sind IMTP®, Intalox® Ultra, Raschig® Super Ring, Cascade Mini Ring® oder Nutter® Ring. Die Trennwandkolonne kann z. B. bei einer Sumpftemperatur von 50 bis 100 °C und bei einem Druck von 4 bis 10 bar betrieben werden. All die im vorliegenden Dokument angegebenen Drücke sind Absolutdrücke.

Die Trennleistung einer Destillationskolonne oder eines Kolonnenabschnitts wird üblicherweise durch die Anzahl der darin vorhandenen theoretischen Trennstufen angegeben. Als theoretische Trennstufe, in der Literatur oftmals auch als "theoretischer Boden" bezeichnet, wird die Kolonneneinheit definiert, die eine Anreicherung an leichter flüchtiger Komponente entsprechend dem thermodynamischen Gleichgewicht zwischen Flüssigkeit und Dampf in einem einzigen Destillationsvorgang bewirkt. Die Trennwandkolonne weist insbesondere 30 bis 100 theoretische Trennstufen oder besonders bevorzugt 50 bis 80 theoretische Trennstufen auf. Die Trennwand erstreckt sich im Allgemeinen über 4 bis 60, vorzugsweise über 6 bis 50 theoretische Trennstufen.

In einer Ausführungsform wird der gereinigte C₄-Schnitt gasförmig aus der Trennwandkolonne als Seitenstrom abgezogen. Die Trennwandkolonne bewirkt so gleichzeitig eine Vorreinigung und Verdampfung des Roh-C₄-Schnitts. In anderen Ausführungsformen wird der flüssig abgezogene gereinigte C₄-Schnitt in einem separaten Verdampfer verdampft.

Den gereinigten C₄-Schnitt unterzieht man wenigstens einer Extraktivdestillation mit einem selektiven Lösungsmittel, wobei man zumindest eine Butane und Butene enthaltende Fraktion und eine Rein-1,3-Butadien-Fraktion erhält. Die Butane und Butene enthaltende Fraktion wird üblicherweise als Raffinat 1 bezeichnet. In der Regel erhält man außerdem wenigstens eine weitere Fraktion, d.h. eine Acetylene-Fraktion, welche die C₄-Acetylene Ethylacetylen und Vinylacetylen, enthält. Das von Acetylenen befreite selektive Lösungsmittel wird vorliegend auch als ausgegastes selektives Lösungsmittel bezeichnet.

Das beim erfindungsgemäßen Verfahren erhaltene Raffinat 1 ist weitgehend frei von C₃-Kohlenwasserstoffen, C₅₊-Kohlenwasserstoff und Karbonylen, die in Downstream-Anlagen auf verschiedene Weise stören können.

Aufgrund der hohen Reaktionsfähigkeit des 1,3-Butadiens bilden sich während der Extraktion und des Ausgasens des selektiven Lösungsmittels unweigerlich reaktive Oligomere und Polymere des 1,3-Butadiens, sowie 4-Vinylcyclohexen (im Folgenden Vinylcyclohexen), das ein Dimerisierungsprodukt von 1,3-Butadien ist. Die 1,3-Butadien-Oligomere und Vinylcyclohexen reichern sich aufgrund ihres gegenüber C₄-Kohlenwasserstoffen höheren Siedepunkts ebenfalls im selektiven Lösungsmittel an. Auch wenn in der Vordestillationskolonne 1,3-Butadien-Polymere und -oligomere sowie Vinylcyclohexen nahezu vollständig entfernt werden, lässt sich ihre Neubildung nicht völlig unterdrücken. Um eine Aufpegelung von Vinylcyclohexen im Verfahren zu vermeiden, wird Vinylcyclohexen vorzugsweise ebenfalls vom selektiven Lösungsmittel desorbiert oder ausgestrippt. Vinylcyclohexen kann bevorzugt über die Acetylene-Fraktion entfernt werden.

Die einfachste Möglichkeit, 1,3-Butadien-Oligomere und -Polymere zu entfernen, besteht darin, eine Lösungsmittelmenge abzustoßen, mit der eine solche Menge an 1,3-Butadien-Oligomeren und -Polymeren entfernt wird, die der eingetragenen Menge entspricht. Vorzugsweise aber wird ein Teilstrom des selektiven Lösungsmittels kontinuierlich oder periodisch einer Aufarbeitung unterzogen, bei der 1,3-Butadien-Oligomere und -Polymere z. B. destillativ entfernt werden. Eine solche Prozessaufbereitungsstufe ist in den Butadien-Extraktionsanlagen ohnehin vorhanden, um aus dem ausgegasten Lösungsmittel verschiedene Chemikalien wie Antischaummittel oder Inhibitoren, Polymere, Oligomere und Zersetzungsprodukte des selektiven Lösungsmittels zu entfernen.

Bei der Extraktivdestillation erhält man eine beladene Lösungsmittel-Fraktion, die 1,3-Butadien und selektives Lösungsmittel enthält. Von der beladenen Lösungsmittel-Fraktion wird Roh-1,3-Butadien desorbiert. Da das ausgegaste selektive Lösungsmittel eine endliche Konzentration an Vinylcyclohexen enthält, kann das Roh-1,3-Butadien eine Konzentration an Vinylcyclohexen aufweisen, die über der typischen Spezifikationsgrenze liegen kann. In einer Ausführungsform wird daher vorgeschlagen, das Roh-1,3-Butadien in einer Rektifikationszone mit flüssigem 1,3-Butadien zu waschen, das durch Kondensation des am Kopf der Rektifikationszone anfallenden Brüdens erhalten wird. Der am Kopf der Rektifikationszone anfallenden Brüden wird in einem Kühler kondensiert. Über einen Rücklaufteiler kann ein Teil des Kondensats als Rücklauf auf die Rektifikationszone gegeben werden, während der andere Teil als Rein-1,3-Butadien abgeführt wird. Durch den Rücklauf werden Vinylcyclohexen und Lösungsmittelreste niedergeschlagen und das am Kopf der Rektifikationszone anfallende Rein-1,3-Butadien erfüllt die Spezifikationskriterien.

Die Rektifikationszone enthält trennwirksame Einbauten, vorzugsweise Trennböden, bevorzugt Sieb-, Glocken- und Ventilböden. Die Rektifikationszone umfasst wenigstens 2, vorzugsweise wenigstens 4, insbesondere 4 bis 8, theoretische Trennstufen. In einer Ausführungsform umfasst die Rektifikationszone 6 bis 10 praktische Böden.

In der Regel wird das erhaltene Rein-1,3-Butadien keiner weiteren Destillation, insbesondere zur Entfernung von 1,2-Butadien und/oder C₅₊-Kohlenwasserstoffen (einschließlich C₄-Oligomeren und -Polymeren), unterworfen.

Typischerweise wird das von der beladenen Lösungsmittel-Fraktion desorbierte Roh-1,3-Butadien, das noch C₄-Acetylene enthalten kann, mit ausgegastem selektivem Lösungsmittel extrahiert. Hierzu bringt man das Roh-1,3-Butadien in einer Nachwaschzone mit ausgegastem selektivem Lösungsmittel in Kontakt. Dabei werden die im Roh-1,3-Butadien noch enthaltenen C₄-Acetylene extrahiert. Aus der Nachwaschzone kann das von C₄-Acetylenen befreite Roh-1,3-Butadien in die vorstehend beschriebene Rektifikationszone geführt werden.

Der Schritt b) kann zwei Extraktivdestillationsschritte umfassen. In einem ersten Abschnitt werden der gereinigte C₄-Schnitt und das selektive Lösungsmittel in eine erste Extraktivdestillationskolonne geführt, worin der gereinigte C₄-Schnitt in zwei Fraktionen zerlegt wird: ein Raffinat 1-Kopfprodukt, das die im selektiven Lösungsmittel weniger löslichen Komponenten Butane und Butene enthält, und das Sumpfprodukt, das das selektive Lösungsmittel mit dem darin gelösten Butadien und C₄-Acetylene, wie Vinylacetylen, enthält, die die löslicheren Komponenten darstellen. Das selektive Lösungsmittel mit dem darin gelösten Butadien und C₄-Acetylenen wird in einen ersten Stripper geleitet, in dem die Butadiene und C₄-Acetylene aus dem selektiven Lösungsmittel gestrippt werden und über Kopf in die zweite Extraktivdestillationskolonne geführt werden. Das ausgestrippte selektive Lösungsmittel wird zurück in die erste Extraktivdestillationskolonne geschickt.

In der zweiten Extraktivdestillationskolonne wird der Butadien-/Acetylene-Strom aus dem ersten Abschnitt wiederum in zwei Fraktionen zerlegt: eine 1,3-Butadien-Kopffraktion und eine Sumpffraktion, die das selektive Lösungsmittel und C₄-Acetylene enthält, die gegenüber 1,3-Butadien im selektiven Lösungsmittel besser löslich sind. Das Sumpfprodukt aus der zweiten Extraktivdestillation wird in einem zweiten Stripper behandelt, in dem ein C₄-Acetylene-reiches Produkt aus dem selektiven Lösungsmittel ausgestrippt wird. In der ersten Extraktivdestillationskolonne und in der zweiten Extraktivdestillationskolonne können das gleiche oder verschiedene selektive Lösungsmittel verwendet werden.

In der bevorzugten Ausführungsform umfasst der Schritt b) eine einstufige Extraktion mit einer fraktionierten Desorption. Dabei werden die im selektiven Lösungsmittel absorbierten Kohlenwasserstoffe in der umgekehrten Reihenfolge ihrer Affinität zum selektiven Lösungsmittel desorbiert.

Den gasförmigen gereinigten C₄-Schnitt bringt man in wenigstens einer Extraktionskolonne mit einem selektiven Lösungsmittel in Kontakt, wobei man eine Butane und Butene enthaltende Kopffraktion und eine 1,3-Butadien, C₄-Acetylene und selektives Lösungsmittel enthaltende Sumpffraktion erhält. Normalerweise bringt man den gasförmigen C₄-Schnitt mit dem selektiven Lösungsmittel in Kontakt, indem man den gasförmigen C₄-Schnitt in wenigstens einem Abschnitt der Extraktionskolonne(n) im Gegenstrom zum selektiven Lösungsmittel führt.

Aus Investitionskostengründen kann es vorteilhaft sein, anstelle einer einzelnen Extraktionskolonne zwei Kolonnen so zu koppeln, dass sie thermodynamisch in der Summe die gleiche Trennstufenzahl wie die Einzelkolonne aufweisen. Die Extraktions- und die Ausgaserkolonne(n) können auch je ganz oder teilweise in einer integrierten Extraktions- und Ausgaserkolonne integriert sein. Der Extraktions- und Ausgaserkolonne können (eine) weitere Extraktionskolonne(n) vorgeschaltet und/oder (eine) weitere Ausgaserkolonne(n) nachgeschaltet sein. Wenn der integrierten Extraktions- und Ausgaserkolonne eine weitere Ausgaserkolonne nachgeschaltet ist, wird im vorliegenden Dokument bei der integrierten Kolonne von einer Extraktions- und Vorausgaserkolonne gesprochen.

Die Prozessparameter, wie Druck, Temperatur und Lösungsmittelverhältnis, stellt man in der Extraktionskolonne so ein, dass diejenigen Komponenten des C₄-Schnittes, für die das selektive Lösungsmittel eine geringere Affinität als für 1,3-Butadien hat, insbesondere die Butane und die Butene, im Wesentlichen in der Gasphase verbleiben, wogegen 1,3-Butadien sowie Acetylene und weitere Kohlenwasserstoffe, für die das selektive Lösungsmittel eine höhere Affinität als für 1,3-Butadien hat, vom selektiven Lösungsmittel im Wesentlichen vollständig absorbiert werden. So erhält man die Butane und Butene enthaltende Kopffraktion und die 1,3-Butadien, C₄-Acetylene, und selektives Lösungsmittel enthaltende Sumpffraktion. Die Kopffraktion wird üblicherweise als Raffinat 1 bezeichnet. Die Extraktionskolonne kann z. B. bei einer Temperatur von 20 bis 80 °C und bei einem Druck von 3 bis 6 bar betrieben werden Vorteilhaft wird der Druck so gewählt, dass der Kopfkondensator der Extraktionskolonne mit einem leicht verfügbaren Kühlmittel, wie Kühlwasser, betrieben werden kann.

Die Sumpffraktion enthält neben dem Lösungsmittel und 1,3-Butadien in der Regel weitere Kohlenwasserstoffe, für die das selektive Lösungsmittel eine höhere Affinität als für 1,3-Butadien hat, wie z. B. C₄-Acetylene.

In einer Ausführungsform leitet man die Sumpffraktion der Extraktionskolonne in eine Extraktions- und Vorausgaserkolonne. Der obere Teil der Extraktions- und Vorausgaserkolonne wirkt als Abtriebsteil, in dem die im Lösungsmittel noch gelösten Butane und Butene sowie andere Leichtsieder ausgetrieben und über Kopf abgezogen werden können. Das Kopfprodukt der Extraktions- und Vorausgaserkolonne kann wieder der Extraktionskolonne zugeführt werden. Das im Sumpf der Extraktions- und Vorausgaserkolonne desorbierte Roh-1,3-Butadien, das neben 1,3-Butadien geringe Mengen C₄-Acetylene und Vinylcyclohexen enthalten, kann als Seitenabzug aus der Extraktions- und Vorausgaserkolonne abgezogen werden. Vorentgastes Lösungsmittel, das noch verschiedene C₄-Komponenten, wie Vinylacetylen, enthält, fällt im Sumpf der Extraktions- und Vorausgaserkolonne an. Die Extraktions- und Vorausgaserkolonne kann z.B. bei einer Sumpftemperatur von 20 bis 80 °C und bei einem Druck von 3 bis 6 bar betrieben werden.

Vorzugsweise leitet man das im Sumpf der Extraktions- und Vorausgaserkolonne anfallende vorausgegaste Lösungsmittel in eine Ausgaserkolonne, in der man weitere Kohlenwasserstoffe, für die das selektive Lösungsmittel eine höhere Affinität als für 1,3-Butadien hat, z. B. C₄-Acetylene, desorbiert. Vorzugsweise wird zusammen mit C₄-Acetylenen auch Vinylcyclohexen desorbiert. Die Ausgaserkolonne kann z. B. bei einer Temperatur von 120 bis 200 °C und bei einem Druck von 1,2 bis 6 bar betrieben werden.

Vorzugsweise zieht man das C₄-Acetylene und Vinylcyclohexen enthaltende Gas als Seitenabzug aus der Ausgaserkolonne ab. Beispielsweise kann man das als Seitenabzug aus der Ausgaserkolonne abgezogene, C₄-Acetylene enthaltende Gas in einem Acetylenwäscher mit Wasser waschen, um selektives Lösungsmittel zurückzugewinnen. Der Acetylenwäscher kann als Seitenkolonne der Ausgaserkolonne ausgebildet sein. Das Waschwasser kann in den Lösungsmittelkreislauf recycliert werden, z. B. in die Ausgaserkolonne und/oder die Extraktions- und Vorausgaserkolonne. Wasserdampf, der vom gewaschenen C₄-Acetylene enthaltenden Gas mitgeführt wird, kann auskondensiert und ganz oder teilweise in den Acetylenwäscher zurückgeführt werden.

Um denjenigen Teil des 1,3-Butadiens rückzugewinnen, der erst in der Ausgaserkolonne desorbiert wird, kann man das Kopfprodukt der Ausgaserkolonne komprimieren und in die Extraktions- und Vorausgaserkolonne zurückführen. Geeigneterweise wird das Kopfprodukt der Ausgaserkolonne vordem Komprimieren gekühlt, z. B. mittels eines Direktkühlers.

Alternativ betreibt man die Ausgaserkolonne auf gleichem Druckniveau wie die Extraktions- und Vorausgaserkolonne, so dass man das Kopfprodukt der Ausgaserkolonne ohne Druckerhöhung in die Extraktions- und Vorausgaserkolonne zurückführen kann. Da beim erfindungsgemäßen Verfahren polymerisierbare C₅-Diene wie Isopren oder cis-2-Pentadien bereits bei der Vordestillation entfernt werden, besteht keine Foulinggefahr in der Ausgaserkolonne.

Am Sumpf der Ausgaserkolonne erhält man ein ausgegastes selektives Lösungsmittel, das zunächst zur Wärmerückgewinnung eingesetzt werden kann und nach einer Schlusskühlung teilweise in die Extraktionskolonne und teilweise in die nachstehend beschriebene Nachwaschzone zurückgeführt wird.

Das desorbierte Roh-1,3-Butadien aus der Sumpffraktion der Extraktions- und Vorausgaserkolonne, das noch verschiedene C₄-Komponenten und Vinylcyclohexen enthalten kann, behandelt man vorzugsweise nacheinander in einer Nachwaschzone mit ausgegastem selektiven Lösungsmittel und in einer Rektifikationszone mit flüssigem 1,3-Butadien, das durch Kondensation des am Kopf der Rektifikationszone anfallenden Brüdens erhalten wird. In der Nachwaschzone bringt man das Roh-1,3-Butadien mit ausgegastem selektivem Lösungsmittel in Kontakt. Dabei werden die im Roh-1,3-Butadien noch enthaltenen C₄-Acetylene extrahiert. Das aus der Nachwaschzone ablaufende Lösungsmittel kann in die Extraktions- und Vorausgaserkolonne geleitet werden.

In der Rektifikationszone wird das von Acetylenen befreite Roh-1,3-Butadien in einer mit flüssigem 1,3-Butadien gewaschen, das durch Kondensation des am Kopf der Rektifikationszone anfallenden Brüdens erhalten ist. Der am Kopf der Rektifikationszone anfallenden Brüden wird in einem Kühler kondensiert. Über einen Rücklaufteiler kann ein Teil des Kondensats als Rücklauf auf die Rektifikationszone gegeben werden, während der andere Teil als Rein-1,3-Butadien abgeführt wird. Durch den Rücklauf werden Vinylcyclohexen und Lösungsmittelreste niedergeschlagen und das am Kopf der Rektifikationszone anfallende Rein-1,3-Butadien erfüllt die Spezifikationskriterien.

Die Nachwaschzone und die Rektifikationszone können von einer separaten Kolonne gebildet sein. In einer geeigneten Ausführungsform sind die Nachwaschzone und die Rektifikationszone in einem durch eine im Wesentlichen in Längsrichtung der Kolonne verlaufende Trennwand abgetrennten oberen Abschnitt der Extraktions- und Vorausgaserkolonne ausgebildet. In einem oberen Bereich der Extraktions- und Vorausgaserkolonne ist dann eine Trennwand in Kolonnenlängsrichtung angeordnet, unter Ausbildung einer Nachwasch- und Rektifikationszone im oberen Abschnitt und eines der Desorption dienenden unteren Abschnitts, der sich unten an die Trennwand anschließt. Vorzugsweise ist die Trennwand außermittig angeordnet, und zwar dergestalt, dass die Querschnittsfläche der Nachwasch- und Rektifikationszone kleiner ist gegenüber der Querschnittsfläche der Extraktionszone.

Der Wassergehalt des am Kopf der Rektifikationszone kondensierten Rein-1,3-Butadiens entspricht der physikalischen Löslichkeit von Wasser in 1,3-Butadien. In den meisten Fällen sind niedrigere Wassergehalte gewünscht oder vorgegeben. In diesen Fällen kann man das Rein-1,3-Butadien in einem weiteren Schritt einer Wasserentfernung unterziehen.

Zur Wasserentfernung kann man das Rein-1,3-Butadien z. B. abkühlen; dadurch sinkt die physikalische Löslichkeit von Wasser in 1,3-Butadien und das nun nicht mehr vollständig lösliche Wasser bildet eine eigene Phase, die abgetrennt werden kann. Alternativ kann Wasser in einer Strippkolonne entfernt werden. In der Strippkolonne wird das Rein-1,3-Butadien mittels eines Strippdampfes gestrippt, der durch Teilverdampfung des Rein-1,3-Butadiens im Sumpf der Strippkolonne erhalten ist. Der am Kopf der Strippkolonne anfallende Brüden wird kondensiert und das Kondensat einer Phasentrennung in eine wässrige und eine organische Phase unterworfen. Die organische Phase kann als Rücklauf in die Strippkolonne geleitet werden. Entwässertes Rein-1,3-Butadien wird über Sumpf abgezogen. Andere Verfahren zur Entwässerung des Rein-1,3-Butadiens, wie Umkehrosmose oder Adsorption, sind ebenfalls vorstellbar.

Der am Kopf der Rektifikationszone nicht kondensierte Teil des Brüdens wird als Auslassstrom abgezogen. Der Auslassstrom besteht im Wesentlichen aus 1,3-Butadien mit Spuren von Wasserdampf, Sauerstoff und Inertgasen. Der Auslassstrom dient zum Ausschleusen von Sauerstoff und Inertgasen. So kann der Sauerstoffgehalt in der Extraktions- und Vorausgaserkolonne unter die Nachweisgrenze üblicher Sauerstoffdetektoren gedrückt werden. Die Anwesenheit geringer Mengen an molekularem Sauerstoff im unteren ppm-Bereich oder darunter wurde als eine Hauptursache für die unerwünschte Bildung von Butadien-Polymeren in den Kolonnen und Anlagenteilen erkannt.

Gemäß einer bevorzugten Ausführungsform des erfindungsgemäßen Verfahrens führt man den Auslassstrom aus der Rektifikationszone zusammen mit dem Brüden der Vordestillationskolonne durch den Kopfkondensator der Vordestillationskolonne. Der Auslassstrom aus der Rektifikationszone kann z. B. am Kopf der Vordestillationskolonne oder in die Zufuhrleitung zum Kondensator eingeführt werden. Im Auslassstrom aus der Rektifikationszone enthaltenes 1,3-Butadien geht bei dieser Verfahrensführung nicht verloren. Der Kopfkondensator der Vordestillationskolonne wird so betrieben, dass C₃-Kohlenwasserstoffe als Kopfstrom abgetrennt werden, höhere Kohlenwasserstoffe dagegen kondensiert und als Rücklauf auf die Vordestillationskolonne gegeben werden. Das 1,3-Butadien kondensiert daher im Kopfkondensator der Vordestillationskolonne per Massenbilanz praktisch vollständig, strömt in die Vordestillationskolonne und bildet einen Teil des daraus als Seitenstrom abgezogenen gasförmigen C₄-Schnitts.

Gemäß einer weiteren bevorzugten Ausführungsform sieht man außerdem einen gasförmigen Auslassstrom aus der Extraktionskolonne vor. Vorzugsweise ist der Auslass am oder hinter dem Kondensator, z. B. am Destillatsammler, angeordnet, d.h. man leitet die verbleibenden Gasbestandteile aus, die im Kopfkondensator der Extraktionskolonne nicht kondensiert werden. Diese nicht kondensierten Bestandteile bestehen im Wesentlichen aus Butanen, Butenen, Inertgasen wie Stickstoff und untergeordneten Mengen an molekularem Sauerstoff. Mit Vorteil kann man den Auslassstrom aus der Extraktionskolonne verwenden, um das C₄-Acetylene enthaltende Gas zu verdünnen, das in der Ausgaserkolonne desorbiert wird.

Der Roh-C₄-Schnitt enthält zumindest 1,3-Butadien und Butene. In den meisten Fällen enthält der Roh-C₄-Schnitt 1,3-Butadien, Butane, Butene und C₄-Acetylene. In vielen Fällen enthält der Roh-C₄-Schnitt 1,3-Butadien, 1,2-Butadien, Butane, Butene und C₄-Acetylene, C₃-Kohlenwasserstoffe und C₅₊-Kohlenwasserstoffe. Unter C₅₊-Kohlenwasserstoffen werden vorliegend Kohlenwasserstoffe mit fünf oder mehr Kohlenstoffen verstanden.

Bei dem Roh-C₄-Schnitt handelt es sich z. B. um einen Roh-C₄-Schnitt aus einem Naphtha Cracker.

Ein typischer Roh-C₄-Schnitt aus einem Naphtha Cracker weist die folgende Zusammensetzung in Gewichtsprozenten auf:

| | |
|---|---|
| Propan | 0 - 0,5 |
| Propen | 0 - 0,5 |
| Propadien | 0 - 0,5 |
| Propin | 0 - 0,5 |
| n-Butan | 3 - 10 |
| i-Butan | 1 - 3 |

| | |
|---|---|
| 1-Buten | 10 - 20 |
| i-Buten | 10 - 30 |
| trans-2-Buten | 2 - 8 |
| cis-2-Buten | 2 - 6 |
| 1,3-Butadien | 15 - 85 |
| 1,2-Butadien | 0,1 - 1 |
| Ethylacetylen | 0,1 - 2 |
| Vinylacetylen | 0,1 - 3 |
| C₅-Kohlenwasserstoffe | 0 - 0,5 |

Roh-C₄-Schnitte aus Naphtha-Crackern enthalten somit überwiegend Butane, Butene und 1,3-Butadien. Darüber hinaus sind geringe Mengen an sonstigen Kohlenwasserstoffen enthalten. C₄-Acetylene sind gelegentlich bis zu einem Anteil von 5 Gew.-% und häufig bis zu 2 Gew.-% enthalten.

Als selektive Lösungsmittel kommen generell Substanzen oder Gemische in Frage, die einen höheren Siedepunkt als das aufzutrennende Gemisch sowie eine größere Affinität zu konjugierten Doppelbindungen und Dreifachbindungen als zu einfachen Doppelbindungen sowie Einfachbindungen aufweisen, bevorzugt dipolare, besonders bevorzugt dipolar-aprotische Lösungsmittel. Aus apparatetechnischen Gründen werden wenig oder nicht korrosive Substanzen bevorzugt. Geeignete selektive Lösungsmittel für das erfindungsgemäße Verfahren sind zum Beispiel Nitrile wie Acetonitril, Propionitril, Methoxypropionitril, Ketone wie Aceton, Furfurol, N-alkylsubstituierte niedere aliphatische Säureamide, wie Dimethylformamid, Diethylformamid, Dimethylacetamid, Diethylacetamid, N-Formylmorpholin, N-alkylsubstituierte cyclische Säureamide (Lactame) wie N-Alkylpyrrolidone, insbesondere N-Methylpyrrolidon (NMP). Im Allgemeinen werden N-alkylsubstituierte niedere aliphatische Säureamide oder N-alkylsubstituierte cyclische Säureamide verwendet. Besonders vorteilhaft sind Dimethylformamid, Acetonitril, Furfurol und insbesondere N-Methylpyrrolidon.

Es können jedoch auch Mischungen dieser Lösungsmittel untereinander, zum Beispiel von N-Methylpyrrolidon mit Acetonitril, Mischungen dieser Lösungsmittel mit Co-Lösungsmitteln wie Wasser, Alkoholen, insbesondere solchen mit 5 oder weniger Kohlenstoffatomen, wie Methylalkohol, Ethylalcohol, n-Propylalkohol, Isopropylalkohol, n-Butylalkohol, Isobutylalkohol, sec-Butylalkohol, tert-Butylalkohol, n-Pentylalkohol, oder alicyclische Alkohole wie Cyclopentanol, Diolen, wie Ethylenglycol, und/oder tert.-Butylether, zum Beispiel Methyl-tert-butylether, Ethyl-tert.-butylether, Propyl-tert-butylether, n- oder iso-Butyl-tert-butylether eingesetzt werden.

Gemäß einer bevorzugten Ausführungsform des erfindungsgemäßen Verfahrens enthält das selektive Lösungsmittel wenigstens 80 Gew.-% N-Methylpyrrolidon. Bevorzugt enthält das selektive Lösungsmittel 85 bis 95 Gew.-% NMP und 5 bis 15 Gew.-% Wasser. Besonders geeignet ist N-Methylpyrrolidon, bevorzugt in wässriger Lösung, insbesondere mit 7 bis 9 Gew.-% Wasser, besonders bevorzugt mit 8,3 Gew.-% Wasser.

Darüber hinaus kann das selektive Lösungsmittel insbesondere noch Hilfsstoffe, wie Inhibitoren oder Antischaummittel, enthalten. Organische Nebenkomponenten können als Verunreinigung enthalten sein.

Die Erfindung wird durch die beigefügte Zeichnung und die nachfolgenden Beispiele näher veranschaulicht.

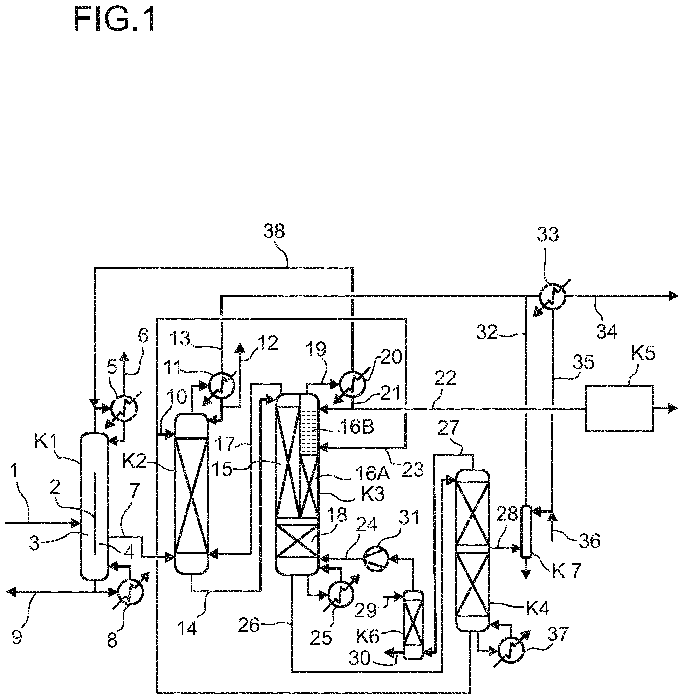

Figur 1 zeigt schematisch eine bevorzugte Anlage zur Durchführung des erfindungsgemäßen Verfahrens.

Ein flüssiger Roh-C₄-Schnitt 1 wird in die Vordestillationskolonne K1 eingeführt. Die Vordestillationskolonne K1 ist in einem mittleren Abschnitt durch eine im Wesentlichen in Längsrichtung der Vordestillationskolonne K1 ausgerichtete Trennwand 2 in einen Zulaufbereich 3 und einen Seitanabzugsbereich 4 unterteilt. Der Zulaufbereich 3 und der Seitenabzugsbereich 4 erstrecken sich in vertikaler Richtung jeweils vom oberen bis zum unteren Ende der Trennwand 2. Aus dem Seitenabzugsbereich 4 zieht man einen gasförmigen C₄-Schnitt 7 ab. Den Brüden der Vordestillationskolonne K1 führt man durch den Kopfkondensator 5. Das darin gebildete Kondensat führt man in die Vordestillationskolonne K1 zurück. Der nicht kondensierte Teil der Brüden bildet den Kopfstrom 6, der als eine Leichtsiederfraktion aus der Vordestillationskolonne K1 abgezogen wird. Aus der Vordestillationskolonne K1 zieht man außerdem eine Hochsiederfraktion als Sumpfstrom 9 ab. Der Sumpf der Vordestillationskolonne K1 wird über Verdampfer 8 beheizt.

Den gasförmigen C₄-Schnitt 7 führt man in einen unteren Abschnitt einer Extraktionskolonne K2 ein und bringt ihn darin mit einem ausgegasten selektiven Lösungsmittel 10 in Kontakt, das man in einen oberen Abschnitt der Extraktionskolonne K2 einspeist. Durch Kondensation des Brüdens der Extraktionskolonne K2 in Kondensator 11 erhält man eine Butane und Butene enthaltende Kopffraktion 12 (sogenanntes Raffinat I) sowie einen nicht kondensierten Anteil 13 des Brüdens. Der Strom 13 dient als Verdünnungsgas für die Acetylene 32. Gleichzeitig ist der Strom 13 Auslassstrom für die Extraktionskolonne K2, um den Gehalt an molekularem Sauerstoff im Gasraum der Extraktionskolonne K2 zu kontrollieren. Man erhält außerdem eine Sumpffraktion 14, die im Wesentlichen aus selektivem Lösungsmittel besteht, in dem neben 1,3-Butadien Vinylacetylen, Ethylacetylen und Vinylcyclohexen sowie Butane und Butene gelöst sind.

Die Sumpffraktion 14 führt man in einen oberen Abschnitt 15 einer Extraktions- und Vorausgaserkolonne K3. Durch eine im Wesentlichen in Längsrichtung der Kolonne verlaufende Trennwand sind vom oberen Abschnitt der Extraktions- und Vorausgaserkolonne K3 eine Nachwaschzone 16A und eine Rektifikationszone 16B abgetrennt. Ein wesentlicher Teil des Roh-1,3-Butadiens wird in dem unteren Abschnitt 18 der Extraktions- und Vorausgaserkolonne K3 desorbiert. Das Roh-1,3-Butadien bringt man in der Nachwaschzone 16A mit über den Seitenzulauf 23 zugeführtem ausgegastem selektivem Lösungsmittel in Kontakt, um C₄-Acetylene abzutrennen. Oberhalb der Nachwaschzone 16A ist die Rektifikationszone 16B angeordnet. Die Rektifikationszone 16B umfasst eine Anzahl an Trennböden. Durch Kondensation des Brüdens 19 in Kondensator 20 erhält man flüssiges Rein-1,3-Butadien 21 sowie einen nicht kondensierten Anteil 38 des Rektifikationsbrüdens. Einen Teil des Kondensats 21 des 1,3-Butadiens führt man als Rücklauf in die Rektifikationszone zurück. Das 1,3-Butadien wird in dieser mit zurücklaufendem Kondensat in Kontakt gebracht, um Schwersieder wie Vinylcyclohexen und Reste des selektiven Lösungsmittels abzutrennen. Den anderen Teil 22 des 1,3-Butadien-Kondensats führt man als Rein-1,3-Butadien der fakultativen Wasserabtrennung K5 zu. Der Sumpf der Extraktions- und Vorausgaserkolonne K3 wird über den Verdampfer 25 beheizt. Den nicht kondensierten Anteil 38 des Rektifikationsbrüdens führt man in den Kopfkondensator 5 der Vordestillationskolonne K1 zurück.

Ein am Kopf der Extraktions- und Vorausgaserkolonne K3 aus Abschnitt 15 ausgeleitetes, Butane und Butene enthaltendes Gas 17 führt man in einen unteren Abschnitt der Extraktionskolonne K2 zurück. Thermodynamisch entsprechen der Abschnitt 15 der Extraktions- und Vorausgaserkolonne K3 und die Extraktionskolonne K2 zusammen einer einzigen Extraktionskolonne, welche aus Gründen der Kolonnenhöhe vertikal zweigeteilt wurde.

Vorausgegastes Lösungsmittel aus dem Sumpf der Extraktions- und Vorausgaserkolonne K3 wird als Strom 26 weiter in eine Ausgaserkolonne K4 geleitet. In der Ausgaserkolonne K4 desorbiert weiteres Roh-1,3-Butadien, das man in den unteren Abschnitt der Extraktions- und Vorausgaserkolonne K3 führt. In der dargestellten Ausführungsform wird das in der Ausgaserkolonne K4 desorbierte Roh-1,3-Butadien über Leitung 27, die fakultative Anordnung von Direktkühler K6 und Kompressor 31, und Leitung 24 in den unteren Abschnitt der Extraktions- und Vorausgaserkolonne K3 geführt. Über die Leitungen 29 und 30 wird Kühlmedium zu- und abgeführt. Als Kühlmedium dient Eigenkondensat, das im Wesentlichen aus Komponenten des selektiven Lösungsmittel, z. B. Wasser und NMP, besteht. Der Sumpf der Ausgaserkolonne K4 wird über Verdampfer 37 beheizt.

Ein C₄-Acetylene und Vinylcyclohexen sowie 1,3-Butadien und Komponenten des selektiven Lösungsmittel, z. B. Wasser und NMP, enthaltendes Gas 28 zieht man als Seitenabzug aus der Ausgaserkolonne K4 ab. Das C₄-Acetylene und Vinylcyclohexen enthaltendes Gas 28 wird im Acetylenwäscher K7 mit Wasser gewaschen, das über Leitung 36 herangeführt wird. Der am Kopf des Acetylenwäschers K7 erhaltene Strom 32 wird mit dem Auslassstrom 13 der Extraktionskolonne K2 verdünnt. Kondensierbare Bestandteile (im Wesentlichen Wasser und geringe Mengen Vinylcyclohexen) werden im Kondensator 33 kondensiert und können als Rücklauf 35 auf den Acetylenwäscher K7 gegeben werden; der Ablauf aus dem Acetylenwäscher K7 ist im Wesentlichen Prozessabwasser, das einer Phasentrennung unterworfen und entsorgt werden kann. Nicht kondensierte Bestandteile werden als Strom 34 (verdünnter Acetylene-Strom) ausgeschleust.

In der fakultativen Wasserabtrennen K5, die z. B. als Strippkolonne ausgestaltet ist, trennt man vom 1,3-Butadien 21 eine Wasserphase ab.

### Beispiel 1

Das erfindungsgemäße Verfahren wurde auf Grundlage einer Anlage gemäß Figur 1 simuliert. Für die Simulationsrechnung wurde die BASF-eigene Software Chemasim verwendet; bei Verwendung kommerziell erhältlicher Software wie Aspen Plus (Hersteller: AspenTech, Burlington/Massachusetts, USA) oder PRO II (Fullerton, USA) würde man vergleichbare Ergebnisse erhalten. Der Parametersatz beruhte auf umfangreichen Gleichgewichtsmessungen, Untersuchungen an Laborkolonnen und Betriebsdaten verschiedener Anlagen. Die Zielspezifikation für das Rein-1,3-Butadien war: Mindestens 99,5 % 1,3-Butadien, maximal 20 ppm 1,2-Butadien, maximal 20 ppm Acetylene.

Ausgegangen wurde von einem Roh-C₄-Schnitt enthaltend 1300 ppm C₃-Kohlenwasserstoffe, 2,0 % n-Butan, 0,6 % iso-Butan, 19,0 % n-Buten, 28,3 % iso-Buten, 5,5 % trans-2-Buten, 4,4 % cis-2-Buten, 39,0 % 1,3-Butadien, 0,2 % 1,2-Butadien, 1200 ppm 1-Butin, 4500 ppm Vinylacetylen und je 3000 ppm C₅-Kohlenwasserstoffe. In Tabelle 1 sind die Massenströme und Zusammensetzungen relevanter Ströme zusammengefasst. Die Bezeichnungen der Ströme in der Tabelle beziehen sich auf die Bezugszeichen der Figur 1.

**Tabelle 1**

| **Beispiel** 1 | Strom | | | |
|---|---|---|---|---|
| | 1 | 7 | 9 | 22 |
| Massenstrom [kg/h] | 32000 | 31585 | 550 | 12667 |
| 1,2-Butadien [Gew.-%] | 0,15 | 0,0012 | 8,66 | 0,0019 |
| Acetylene [Gew.-%] | 0,57 | 0,57 | 0,36 | 0,02 |
| C₅₊-Komponenten [Gew.-%] | 0,30 | 0,002 | 45,00 | |
| 1,3-Butadien [Gew.-%] | 39,0 | 40,2 | 20,0 | 99,53 |

Über den einzigen Hochsiederauslassstrom 9 ergibt sich ein Verlust an 1,3-Butadien von etwa 7 kg/h. In der Vordestillationskolonne wird sowohl der Gehalt an C₅-Komponenten als auch der Gehalt an 1,2-Butadien als die am schwierigsten abzutrennenden Hochsieder um etwa 99 % reduziert.

### Vergleichsbeispiel

Es wurde ein Verfahren des Stands der Technik simuliert. Die Zusammensetzung des Roh-C₄-Schnitts und die Zielspezifikation für das Rein 1,3-Butadien stimmten mit Beispiel 1 überein. Die zugrunde gelegte Anlage wies an Stelle der mit der Trennwand 2 versehenen Vordestillationskolonne K1 eine vorgeschaltete Destillationskolonne gemäß WO 2013/083536 A1 auf, der der flüssige Roh-C₄-Schnitt zugeführt wurde. Außerdem wies die Anlage des Vergleichsbeispiels eine Reindestillationskolonne auf.

In Tabelle 2 sind die Massenströme und Zusammensetzungen relevanter Ströme zusammengefasst.

**Tabelle 2**

| **Vergleichsbeispiel 2** | Strom | | | |
|---|---|---|---|---|
| | Roh-C₄ fl.¹ | Roh-C₄ ger.² | Sumpf Dest.³ | Sumpf Rein⁴ |
| Massenstrom [kg/h] | 32000 | 31743 | 110 | 69 |
| 1,2-Butadien [Gew.-%] | 0,15 | 0,14 | 40,00 | 40,00 |
| Acetylene [Gew.-%] | 0,57 | 0,57 | 0,21 | 0,18 |
| C₅₊-Kom ponenten [Gew.-%] | 0,30 | 0,13 | 8,38 | 30,18 |
| 1,3-Butadien [Gew.-%] | | | 29,88 | 14,88 |

| | | | | |
|---|---|---|---|---|
| 1 flüssiger Roh-C₄-Schnitt (Bezugszeichen 1 in Fig. 1 der WO 2013/083536 A1) 2 dampfförmiger gereinigter Roh-C₄-Schnitt (Bezugszeichen 4 in Fig. 1 der WO 2013/083536 A1) 3 an der vorgeschalteten Destillationskolonne anfallender Sumpfstrom (Bezugszeichen 3 in Fig. 1 der WO 2013/083536 A1) 4 bei der Reindestillation anfallender Sumpfstrom | | | | |

Bei diesem Verfahren fallen zwei C₅₊-haltige Hochsiederströme an, die verworfen werden: Der Sumpfstrom der vorgeschalteten Destillationskolonne und der bei der Reindestillation anfallende Sumpfstrom. Es ergibt sich ein Verlust an 1,3-Butadien von etwa 43 kg/h. In der vorgeschalteten Destillationskolonne des Vergleichsbeispiels 2 wurde nur ein geringerer Anteil der C₅₊-Komponenten abgetrennt (55 %). Über den dampfförmigen gereinigten Roh-C₄-Schnitt wurden die C₅₊-Komponenten zu einem weitaus höheren Anteil in die Extraktionskolonne verschleppt. Darüber hinaus wurde in der vorgeschalteten Destillationskolonne des Vergleichsbeispiels 2 ein geringerer Anteil an 1,2-Butadien abgetrennt (75 %). Demzufolge ist in dem Vergleichsbeispiel eine weitere Abtrennung von 1,2-Butadien in einer Reinkolonne erforderlich, um eine Spezifikation von 20 ppm 1,2-Butadien zu erreichen. Diese zusätzliche nachträgliche Abtrennung ist mit dem erfindungsgemäßen Verfahren nicht mehr nötig.

## Patentansprüche

1. Verfahren zur Gewinnung von Rein-1,3-Butadien aus einem Roh-C₄-Schnitt, welches Rein-1,3-Butadien einen vorgegebenen Höchstgehalt an wenigstens einem Leichtsieder und einen vorgegebenen Höchstgehalt an 1,2-Butadien, jeweils bezogen auf 1,3-Butadien, aufweist, wobei man
a) vom Roh-C₄-Schnitt destillativ eine Leichtsiederfraktion und eine Hochsiederfraktion abtrennt, wobei man einen gereinigten C₄-Schnitt erhält, dessen Gehalt an dem wenigstens einen Leichtsieder, bezogen auf 1,3-Butadien, gleich oder niedriger ist als der vorgegebene Höchstgehalt an dem wenigstens einen Leichtsieder und dessen Gehalt an 1,2-Butadien, bezogen auf 1,3-Butadien, gleich oder niedriger ist als der vorgegebene Höchstgehalt an 1,2-Butadien;
b) den gereinigten C₄-Schnitt wenigstens einer Extraktivdestillation mit einem selektiven Lösungsmittel unterzieht, wobei man zumindest eine Butane und Butene enthaltende Fraktion und eine Rein-1,3-Butadien-Fraktion erhält.

2. Verfahren nach Anspruch 1, wobei der wenigstens eine Leichtsieder Propin ist und der vorgegebene Höchstgehalt an Propin 20 ppm beträgt, bezogen auf 1,3-Butadien.

3. Verfahren nach einem der vorhergehenden Ansprüche, wobei der vorgegebene Höchstgehalt an 1,2-Butadien 25 ppm beträgt, bezogen auf 1,3-Butadien.

4. Verfahren nach einem der vorhergehenden Ansprüche, wobei man
- bei der Extraktivdestillation eine beladene Lösungsmittel-Fraktion erhält, die 1,3-Butadien und selektives Lösungsmittel enthält, und von der beladenen Lösungsmittel-Fraktion Roh-1,3-Butadien desorbiert,
- das Roh-1,3-Butadien in einer Rektifikationszone, die wenigstens 2 theoretische Trennstufen umfasst, mit flüssigem 1,3-Butadien wäscht, das durch Kondensation des am Kopf der Rektifikationszone anfallenden Brüdens erhalten ist, wobei man Rein-1,3-Butadien erhält.

5. Verfahren nach Anspruch 4, wobei man aus der beladenen Lösungsmittel-Fraktion außerdem eine Acetylene-Fraktion desorbiert, wobei man ein ausgegastes selektives Lösungsmittel erhält.

6. Verfahren nach Anspruch 5, wobei man das Roh-1,3-Butadien vor der Wäsche mit dem flüssigen 1,3-Butadien mit ausgegastem selektiven Lösungsmittel extrahiert.

7. Verfahren nach einem der vorhergehenden Ansprüche, wobei man den Roh-C₄-Schnitt in eine Vordestillationskolonne mit einer in Längsrichtung der Kolonne ausgerichteten Trennwand einführt, eine C₃-Kohlenwasserstoffe enthaltende Leichtsiederfraktion als Kopfstrom, eine Hochsiederfraktion als Sumpfstrom und den gereinigten C₄-Schnitt als Seitenstrom abzieht.

8. Verfahren nach Anspruch 6 oder 7, wobei man
- den gereinigten gasförmigen C₄-Schnitt in wenigstens einer Extraktionskolonne mit einem selektiven Lösungsmittel in Kontakt bringt, wobei man eine Butane und Butene enthaltende Kopffraktion und eine 1,3-Butadien und selektives Lösungsmittel enthaltende Sumpffraktion erhält,
- aus der Sumpffraktion Roh-1,3-Butadien desorbiert, wobei man ein vorausgegastes selektives Lösungsmittel erhält,
- aus dem vorausgegasten selektiven Lösungsmittel Acetylene desorbiert, wobei man ein ausgegastes selektives Lösungsmittel erhält,
- das Roh-1,3-Butadien in einer Nachwaschzone mit ausgegastem selektiven Lösungsmittel extrahiert, und
- das extrahierte Roh-1,3-Butadien in einer Rektifikationszone mit flüssigem 1,3-Butadien wäscht, wobei man Rein-1,3-Butadien erhält.

9. Verfahren nach Anspruch 8, wobei die Rektifikationszone oberhalb der Nachwaschzone in einer Kolonne oder einem Kolonnenabschnitt angeordnet ist.

10. Verfahren nach Anspruch 8 oder 9, wobei man den gereinigten C₄-Schnitt in einer Extraktionskolonne und in einem oberen Abschnitt einer Extraktions- und Vorausgaserkolonne mit dem selektiven Lösungsmittel in Kontakt bringt und das Roh-1,3-Butadien in einem unteren Abschnitt der Extraktions- und Vorausgaserkolonne und einer Ausgaserkolonne von der Sumpffraktion desorbiert.

11. Verfahren nach Anspruch 10, wobei die Nachwaschzone und die Rektifikationszone in einem durch eine im Wesentlichen in Längsrichtung der Kolonne verlaufende Trennwand abgetrennten oberen Abschnitt der Extraktions- und Vorausgaserkolonne ausgebildet sind.

12. Verfahren nach Anspruch 10 oder 11, wobei man die Ausgaserkolonne bei gleichem Druckniveau wie die Extraktions- und Vorausgaserkolonne betreibt und man das Kopfprodukt der Ausgaserkolonne ohne Druckerhöhung in die Extraktions- und Vorausgaserkolonne zurückführt.

13. Verfahren nach einem der Ansprüche 8 bis 12, wobei man den am Kopf der Rektifikationszone nicht kondensierten Teil des Brüdens zusammen mit dem Brüden der Vordestillationskolonne durch den Kopfkondensator der Vordestillationskolonne führt.

14. Verfahren nach einem der vorhergehenden Ansprüche, wobei man aus dem Rein-1,3-Butadien gelöstes Wasser abtrennt, wobei die Wasserabtrennung vorzugsweise mittels Kühlung und Phasentrennung und/oder durch Ausstrippen in einer Strippkolonne erfolgt.

15. Verfahren nach einem der vorhergehenden Ansprüche, wobei das selektive Lösungsmittel wenigstens 80 Gew.-% N-Methylpyrrolidon enthält.

## Claims

1. A process for isolating pure 1,3-butadiene from a crude C₄ fraction, which pure 1,3-butadiene has a prescribed maximum content of at least one low boiler and a prescribed maximum content of 1,2-butadiene, in each case based on 1,3-butadiene, wherein
a) a low boiler fraction and a high boiler fraction are separated off by distillation from the crude C₄ fraction, giving a purified C₄ fraction whose content of the at least one low boiler, based on 1,3-butadiene, is equal to or lower than the prescribed maximum content of the at least one low boiler and whose content of 1,2-butadiene, based on 1,3-butadiene, is equal to or lower than the prescribed maximum content of 1,2-butadiene;
b) the purified C₄ fraction is subjected to at least one extractive distillation using a selective solvent, giving at least a fraction comprising butanes and butenes and a pure 1,3-butadiene fraction.

2. The process according to claim 1, wherein the at least one low boiler is propyne and
the prescribed maximum content of propyne is 20 ppm, based on 1,3-butadiene.

3. The process according to any of the preceding claims, wherein the prescribed maximum content of 1,2-butadiene is 25 ppm, based on 1,3-butadiene.

4. The process according to any of the preceding claims, wherein
- a loaded solvent fraction comprising 1,3-butadiene and selective solvent is obtained in the extractive distillation, and crude 1,3-butadiene is desorbed from the loaded solvent fraction,
- the crude 1,3-butadiene is scrubbed in a rectification zone comprising at least two theoretical plates with liquid 1,3-butadiene which has been obtained by condensation of the vapor obtained at the top of the rectification zone, giving pure 1,3-butadiene.

5. The process according to claim 4, wherein an acetylene fraction is also desorbed from the loaded solvent fraction, giving an outgassed selective solvent.

6. The process according to claim 5, wherein the crude 1,3-butadiene is extracted with degassed selective solvent before the scrub using the liquid 1,3-butadiene.

7. The process according to any of the preceding claims, wherein the crude C₄ fraction is introduced into a predistillation column having a dividing wall oriented in the longitudinal direction of the column, and a low boiler fraction comprising C₃ hydrocarbons is taken off as overhead stream, a high boiler fraction is taken off as bottom stream and the purified C₄ fraction is taken off as side stream.

8. The process according to claim 6 or 7, wherein
- the purified gaseous C₄ fraction is brought into contact with a selective solvent in at least one extraction column, giving an overhead fraction comprising butanes and butenes and a bottom fraction comprising 1,3-butadiene and selective solvent,
- crude 1,3-butadiene is desorbed from the bottom fraction, giving a preoutgassed selective solvent,
- acetylenes are desorbed from the preoutgassed selective solvent, giving an outgassed selective solvent,
- the crude 1,3-butadiene is extracted with outgassed selective solvent in an after-scrubbing zone, and
- the extracted crude 1,3-butadiene is scrubbed with liquid 1,3-butadiene in a rectification zone, giving pure 1,3-butadiene.

9. The process according to claim 8, wherein the rectification zone is arranged above the after-scrubbing zone in a column or a column section.

10. The process according to claim 8or 9, wherein the purified C₄ fraction is brought into contact with the selective solvent in an extraction column and in an upper section of an extraction and preoutgassing column and the crude 1,3-butadiene is desorbed from the bottom fraction in a lower section of the extraction and preoutgassing column and an outgassing column.

11. The process according to claim 10, wherein the after-scrubbing zone and the rectification zone are formed in an upper section of the extraction and preoutgassing column which is separated by a dividing wall running essentially in the longitudinal direction of the column.

12. The process according to claim 10 or 11, wherein the outgassing column is operated at the same pressure level as the extraction and preoutgassing column and the overhead product from the outgassing column is recirculated without increasing the pressure into the extraction and preoutgassing column.

13. The process according to any of claims 8 to 12, wherein the part of the vapor which has not been condensed at the top of the rectification zone is conveyed together with the vapor from the predistillation column through the overhead condenser of the predistillation column.

14. The process according to any of the preceding claims, wherein dissolved water is separated off from the pure 1,3-butadiene
wherein the removal of water is carried out preferably by means of cooling and phase separation and/or by means of stripping in a stripping column.

15. The process according to any of the preceding claims, wherein the selective solvent comprises at least 80% by weight of N-methylpyrrolidone.

## Revendications

1. Procédé pour l'obtention de 1,3-butadiène pur à partir d'une fraction en C₄ brute, qui présente du 1,3-butadiène pur, une teneur maximale prédéterminée en au moins un composé volatil et une teneur maximale prédéterminée en 1,2-butadiène, à chaque fois par rapport au 1,3-butadiène, dans lequel
a) on sépare par distillation une fraction de composés volatils et une fraction de composés non volatils de la fraction en C₄ brute, dans lequel on obtient une fraction en C₄ purifiée dont la teneur en l'au moins un composé volatil, par rapport au 1,3-butadiène, est inférieure ou égale à la teneur maximale prédéterminée en l'au moins un composé volatil et dont la teneur en 1,2-butadiène, par rapport au 1,3-butadiène, est inférieure ou égale à la teneur maximale prédéterminée en 1,2-butadiène ;
b) on soumet la fraction en C₄ purifiée à au moins une distillation extractive avec un solvant sélectif, dans lequel on obtient au moins une fraction contenant des butanes et des butènes et une fraction de 1,3-butadiène pur.

2. Procédé selon la revendication 1, l'au moins un composé volatil étant le propyne et la teneur maximale prédéterminée en propyne étant de 20 ppm, par rapport au 1,3-butadiène.

3. Procédé selon l'une quelconque des revendications précédentes, la teneur maximale prédéterminée en 1,2-butadiène étant de 25 ppm, par rapport au 1,3-butadiène.

4. Procédé selon l'une quelconque des revendications précédentes, dans lequel
- on obtient lors de la distillation extractive une fraction de solvant chargé qui contient du 1,3-butadiène et du solvant sélectif, et on désorbe le 1,3-butadiène brut de la fraction de solvant chargé,
- on lave le 1,3-butadiène brut dans une zone de rectification qui comprend au moins 2 plateaux théoriques, avec du 1,3-butadiène liquide qui est obtenu par condensation des vapeurs produites à la tête de la zone de rectification, dans lequel on obtient du 1,3-butadiène pur.

5. Procédé selon la revendication 4, dans lequel on désorbe en outre une fraction d'acétylènes de la fraction de solvant chargé, dans lequel on obtient un solvant sélectif dégazé.

6. Procédé selon la revendication 5, dans lequel on extrait le 1,3-butadiène pur, avant le lavage avec le 1,3-butadiène liquide, avec du solvant sélectif dégazé.

7. Procédé selon l'une quelconque des revendications précédentes, dans lequel on introduit la fraction en C₄ brute dans une colonne de prédistillation dotée d'une paroi de séparation orientée dans la direction longitudinale de la colonne, on soutire une fraction de composés volatils contenant des hydrocarbures en C₃ en tant que flux de tête, une fraction de composés non volatils en tant que flux de fond et la fraction en C₄ purifiée en tant que flux latéral.

8. Procédé selon la revendication 6 ou 7, dans lequel
- on met en contact la fraction en C₄ purifiée gazeuse avec un solvant sélectif dans au moins une colonne d'extraction, dans lequel on obtient une fraction de tête contenant des butanes et des butènes et une fraction de fond contenant du 1,3-butadiène et du solvant sélectif,
- on désorbe du 1,3-butadiène brut de la fraction de fond, dans lequel on obtient un solvant sélectif prégazé,
- on désorbe des acétylènes du solvant sélectif prégazé, dans lequel on obtient un solvant sélectif dégazé,
- on extrait le 1,3-butadiène brut dans une zone de post-lavage avec du solvant sélectif dégazé, et
- on lave le 1,3-butadiène brut extrait dans une zone de rectification avec du 1,3-butadiène liquide, dans lequel on obtient du 1,3-butadiène pur.

9. Procédé selon la revendication 8, la zone de rectification étant agencée au-dessus de la zone de post-lavage dans une colonne ou une fraction de colonne.

10. Procédé selon la revendication 8 ou 9, dans lequel on met en contact la fraction en C₄ purifiée avec le solvant sélectif dans une colonne d'extraction et dans une partie supérieure d'une colonne d'extraction et de prégazage et on désorbe le 1,3-butadiène brut de la fraction de fond dans une partie inférieure de la colonne d'extraction et de prégazage et d'une colonne de dégazage.

11. Procédé selon la revendication 10, la zone de post-lavage et la zone de rectification étant formées dans une partie supérieure de la colonne d'extraction et de prégazage, séparée par une paroi de séparation s'étendant essentiellement dans la direction longitudinale de la colonne.

12. Procédé selon la revendication 10 ou 11, dans lequel on fait fonctionner la colonne de dégazage à un même niveau de pression que la colonne d'extraction et de prégazage et on renvoie le produit de tête de la colonne de dégazage sans augmentation de pression dans la colonne d'extraction et de prégazage.

13. Procédé selon l'une quelconque des revendications 8 à 12, dans lequel on conduit la partie des vapeurs non condensée à la tête de la zone de rectification ensemble avec les vapeurs de la colonne de prédistillation à travers le condensateur de tête de la colonne de prédistillation.

14. Procédé selon l'une quelconque des revendications précédentes, dans lequel on sépare de l'eau dissoute du 1,3-butadiène pur, la séparation d'eau étant réalisée de préférence au moyen d'un refroidissement et d'une séparation de phase et/ou par strippage dans une colonne de strippage.

15. Procédé selon l'une quelconque des revendications précédentes, le solvant sélectif contenant au moins 80 % en poids de N-méthylpyrrolidone.
